# NEW EUROPEAN PATENT SPECIFICATION

(11) **EP 3 113 784 B3**
(45) Date of publication of this specification: **15.07.2020**
(45) Mention of the grant of the patent: 16.08.2017
(21) Application number: 15711875.3
(22) Date of filing: 05.03.2015
(51) Int. Cl.: A61K 36/899, A61K 36/33, A61P 5/24, A61P 17/10, A61P 17/14, A61P 13/08

(54) **COMPOSITIONS BASED ON PLANT EXTRACTS FOR INHIBITION OF THE 5-ALPHA REDUCTASE**
ZUSAMMENSETZUNGEN AUF BASIS VON PFLANZENEXTRAKTEN ZUR HEMMUNG DER 5-ALPHA-REDUKTASE
COMPOSITIONS À BASE D'EXTRAITS DE PLANTES POUR L'INHIBITION DE LA 5-ALPHA-RÉDUCTASE

(30) Priority: 06.03.2014 IT MI20140351
(43) Date of publication of application: 11.01.2017
(73) Proprietor: Bionap S.r.L., 95032 Belpasso (CT) (IT)
(72) Inventor: BONINA, Andrea Filippo, I-95125 Catania (IT); BONINA, Claudia, I-95125 Catania (IT)
(74) Representative: Calogero, Ida
(86) International application number: PCT/IB2015/051621
(87) International publication number: WO 2015/132755

(56) References cited:
- AUFFENBERG G B ET AL: "Established Medical Therapy for Benign Prostatic Hyperplasia", UROLOGIC CLINICS OF NORTH AMERICA 2009 W.B. SAUNDERS USA, vol. 36, no. 4, November 2009 (2009-11), pages 443-459, XP009179772, ISSN: 0094-0143
- DATABASE WPI Week 201327 Thomson Scientific, London, GB; AN 2013-F48351 XP002728797, & KR 2013 0032420 A (JEJULOVE CO LTD) 2 April 2013 (2013-04-02)
- DATABASE WPI Week 201129 Thomson Scientific, London, GB; AN 2011-C50333 XP002728798, & CN 101 942 381 A (UNIV JIANGSU) 12 January 2011 (2011-01-12)
- DATABASE WPI Week 201082 Thomson Scientific, London, GB; AN 2010-N43412 XP002728799, & KR 2010 0111640 A (COSEED BIOPHARM CO LTD) 15 October 2010 (2010-10-15)
- METCALF B W ET AL: "Inhibitors of steroid 5alpha-reductase in benign prostatic hyperplasia, male pattern baldness and acne", TRENDS IN PHARMACOLOGICAL SCIENCES, ELSEVIER, HAYWARTH, GB, vol. 10, no. 12, 1 December 1989 (1989-12-01), pages 491-495, XP025809437, ISSN: 0165-6147, DOI: 10.1016/0165-6147(89)90048-5 [retrieved on 1989-12-01]

## Description

The present invention refers to nutraceutical, cosmetic or pharmaceutical compositions based on a combination of plant extracts from flowers or fruits of Opuntia ficus indica and Oryza sativa (Black rice) for inhibition of the 5-alpha reductase. In particular, the preparations according to the invention are useful in the prevention or treatment of benign prostatic hypertrophy or hyperplasia, and of androgenic alopecia.

Testosterone is a androgenic hormone synthesized by the Leydig cells of testicles controlled by the hypothalamus and by the anterior pituitary gland (1). Testosterone inside the cells, picked-up by the vascular system, is turned into dehydrotestosterone (DHT) by the 5-α-reductase enzyme. The DHT thus produced is capable to bind to androgenic receptors and to act on transcription and expression of specific nuclear genes. The DHT produced acts on differentiation and growth of the prostatic tissue, on growth of male genitalia, on pubertal growth of hair on the face and on the body and is involved in several diseases such as acne, hirsutism, benign prostatic hypertrophy (BPH), androgenic alopecia (AGA), prostatic cancer (1).

Benign prostatic hypertrophy (BPH) and androgenic alopecia (AGA) are androgen-dependent diseases, in which the transformation of testosterone into DHT by the 5-α-reductase plays a key role (2). In the prostate, the DHT is produced by the action of the 5-α-reductase in its two isoforms (type 1 and 2) and is involved in the growth of the prostatic tissue. In the scalp, the DHT is mainly produced by the 5-α-reductase of the type 2 and is responsible for the miniaturization of the hair bulb (2).

Benign prostatic hypertrophy (BPH) is a pathological condition associated with aging affecting up to 20% of 40 years-old men which may also reach 80% in 70 years-old men (3). Compared with a low mortality (0,35/100.000 people), benign prostatic hypertrophy is a potentially evolving disease implying several lower urinary tract symptoms (LUTS) and which, with the progress of the disease, can negatively affect the quality of life of male subjects (4).

The most common symptoms associated with BPH are divided into the obstructive, mechanical and dynamical, and irritative type of symptoms (5,6). The mechanical obstructive symptoms are determined by the excessive growth of epithelial tissues in the transition zone of the prostate, whereas the dynamical ones derive from the increasing of the smooth muscle tone of the bladder neck, of the prostate and its capsule. Among the obstructive symptoms there are: difficulty in starting to urinate, intermittent urine stream, incomplete bladder emptying, reduction of the urinary stream and effort to urinate. The symptoms defined as of irritative origin are nicturia, urgency to urinate, pollakiuria (urinary frequency), incontinence and burning/pain during urination (3,7). The questionnaire of the International Prostate Symptom Score (IPSS) today is used for the assessment of such symptoms and therefore of the seriousness of the disease.

If not properly treated, benign prostatic hypertrophy can develop over the time, leading to more serious consequences for the health of the patient. Urinary retention can determine the development of prostatitis, due to the growth of bacteria in the bladder residue, and kidney stones due to formation of salts in the post-void residual. Moreover, incomplete emptying of the bladder and chronic urinary retention may significantly compromise the renal function with consequent appearance of obstructive uropathy forms.

Still today, the causes of BPH are not yet completely known. It is possible however to assume that such a disease with aging is correlated to a modification of the hormonal status, in particular to the increasing of dehydrotestosterone (DHT) levels, in the male subject. The DHT is indeed a derivative of testosterone involved in the growth of the prostatic tissues, whose accumulation can thus generate a condition of tissue hypertrophy (8). Which is the cause generating such an increase in production of DHT is still subject-matter of study. The scientific evidence however illustrate how the inhibition of the 5-α-reductase, which is the enzyme responsible for the conversion of testosterone into DHT, is capable to work and control such a disease (8). The pharmacological therapy in the treatment of benign prostatic hypertrophy is based on two possible classes of compounds: the α-blockers and the inhibitors of the 5α-reductase (8,9,10).

The α-blockers (α1-adrenergic receptor antagonists) work by relaxing the smooth muscles of arteries, of the prostate and of the bladder neck, so as to reduce urinary obstruction and promote the increase of the urinary flow rate. Among the most common α-blockers there are: doxazosin, terazosin, alfuzosin, silodosin and tamsulosin. Such medicaments are used in the treatment of the symptomatology of benign prostatic hypertrophy but they do not show any action on development of the disease. An extended use of α-blockers may induce several side-effects such as dizziness, headache, malaise, tiredness and difficulty in breathing. Other effects, less frequently observed, arc orthostatic hypotension and retrograde ejaculation.

The inhibitors of the 5α-reductase work by blocking the synthesis and the production of the enzyme and therefore the conversion of testosterone into DHT. Such compounds (such as finasteride and dutasteride) are capable to induce reduction of the volume of the hypertrophic prostate gland and prevent from progression in the growth, for this such therapies are indicated in the treatment of mild and severe BPH (prostatic volume >40 ml) (9). Also in this case, several side-effects are observed such as decreased libido, gynecomastia, retrograde ejaculation (abnormal ejaculation).

Other pharmacological treatments relate to substances with antimuscarinic (anticholinergic) action and the inhibitors of the 5-phosphodiesterase, but their use is restricted due to low efficacy and numerous side-effects (9).

Only in the cases of absolute failing of the pharmacological therapy (even combined), it is possible to resort to surgical treatment. Such treatments however expose patients to high levels of post-surgery risk in connection with the normal functionality and ejaculatory mechanics. Even the most recent less invasive techniques, such as transurethral needle ablation (TUNA) or laser prostatectomy (TURP), present some limitations and are under experimentation (11,12).

Androgenic alopecia or baldness (AGA) is a dermatological condition that affects both men and women. In the case of men, up to 30% over 30 years of age and up to 50% over 50 years of age are affected by this condition. The AGA can affect with lower incidence even women, although with mild clinical signs in general, such as diffuse thinning of hair on the whole upper part of the scalp (13). The primary causes of androgenic alopecia are not fully known and several factors may work on this condition, such as the age and genetic predisposition. Moreover, there is a certain correlation between occurrence of alopecia and the androgenic activity in the individual, in particular, the production of DHT by part of the 5-α-reductase enzyme. Indeed, the DHT present in the hair follicles is capable to bind to the androgenic receptors and to induce, in the life cycle of the hair, a reduction in the duration of the anagen phase and an extension of the resting phase. As a consequence, the hair will suffer a decrease of their length and of their diameter (miniaturization). After the telagen phase and hair loss, it will be observed a delay in restoring the growth phase and the follicle, as a result, will remain empty for a longer period of time. This will imply a reduction of density of hair and an apparent thinning in the involved zone of the scalp. The recurrence of this condition (miniaturization) in the various life cycles, will lead to the final death of the bulb and irreversible loss of the hair (13).

Minoxidil, taken by topical or systemic route, constitutes one of the most common treatments of androgenic alopecia. Originally used as vasodilator in the treatment of hypertension, Minoxidil seems capable to directly act on proliferation and differentiation of follicular keratinocytes, inducing an extension of the anagen phase. Often used in combination with other active substances, the effect of Minoxidil is however limited over the time after suspension of the therapy. On the other hand, the extended use of this medicament seems to considerably increase the risk of undesired side-effects such as irritation, skin rash, itching also in severe form.

Also the selective inhibitors of the 5-α-reductase, for example finasteride, are used in the systemic therapies of AGA. These therapies present however some disadvantages, such as: long treatment periods required (at least one year), high dosages and therefore its high cost, the side-effects associated with this class of medicaments (gynecomastia, sexual dysfunctions, etc.) (13).

There is therefore the need of alternative treatments of benign prostatic hypertrophy, rather than androgenic alopecia, which do not present the serious side-effects reported in the state of the art.

The authors of the present invention have now found that the combined use of plant extracts of Opuntia Ficus Indica flower and/or fruit and of Oryza sativa (Black rice) entails and anti-proliferative action of the synergistic type on cell lines of benign prostatic hypertrophy. Likewise, this combination of plant extracts shows a proliferative action of the synergistic type on immortalized cellular lines of follicular dermal papillae (used in literature to study the effect on androgenic alopecia). The combination of the two plant extracts did not show any cytotoxicity on normal cell lines, i.e. cell lines of fibroblasts, as it results from the essays of the Trypan blue (data not shown). Opuntia Ficus Indica (Nopal) is a plant belonging to the family of Cactaceae native to Mexico and Southwest of the United States, but also common among the natural plants around the Mediterranean basin. In folk medicine moreover the several parts of the plant find use in numerous applications: fruits are considered astringent and due to their richness in vitamin C they have been used in the past by sailors for the prevention of scurvy; the young cladodes - heated in the oven - are used as emollients, applied in the form of cataplasm; the direct application of the pulp of cladodes on wounds and sores constitutes an excellent anti-inflammatory remedy, restorative and healing on skin wounds and skin ulcers; decoction of flowers has diuretic properties and in Israel it is considered a natural remedy for the treatment of benign prostatic hypertrophy (BPH).

In the study carried out by Jonas et al. (14), it has been observed that the extracts from flowers of the cactus variety obtained with aqueous and organic solvent are capable to act as inhibitors both of the 5-α-reductase enzyme and of aromatase.

Ammar et al. (15) have achieved a quali-quantitative characterization of the compounds contained in the flowers of Opuntia ficus indica. According to what has been disclosed by the authors, the extract in hexane of the flowers is mainly constituted by derivatives of carboxylic acids (29-97%); terpenes (0,2-57%), esters (0,2-27%) and, in lower amount, by alcoholic compounds (<1,8%). Recently, a particular attention has been given to the flavonoid type compounds present in such a plant matrix. De Leo et al (16) have identified, in the methanolic extract from flowers of Opuntia ficus indica, the presence of glycosylated derivatives of kaempferol, of quercetin and isorhamnetin, with a total contents reaching 81,75 mg/1 g of fresh flower.

Among these compounds, the isorhamnetin 3-O-robinobioside is the most prevalent compound, representing about 52% with respect to the total flavonoid contents.

Black rice (Oryza sativa L.) is a type of rice whose grain presents a typical pericarp (seed shell) of black colour.

Nowadays the Black rice is distributed throughout the world market and it is also cultivated in Italy. Indeed, from the crossbreed of Black rice with Italian rice lines, it was obtained a hybrid (Venus rice) which maintains the peculiarities of the Chinese rice, but capable to adapt to our own climates.

In China the Black rice is considered a valuable food product for the richness in nutrients present (proteins and mineral salts such as iron, manganese, selenium), but mostly for its beneficial and healthy properties.

The Black rice is indeed used in folk medicine in the treatment of anaemia, in strengthening the kidney function, in promoting blood circulation and in the treatment of diabetes (17). Such properties are nowadays mainly ascribed to the anti-oxidant active substances present in the pericarp and that confer the peculiar colour thereto: the anthocyanins. Recent studies carried out by Min-Kyoung et al. (18) have shown that the Black rice contains three anthocyanoside species: cyanidin-3-glucoside; delphinidin-3-glucoside and petudin-3-glucoside. Among these, however cyanidin-3-glucoside presents as the compound provided in prevailing amount so that we can consider the Black rice a natural source of such an anthocyanoside compound (18).

A subject-matter of the present invention is a nutraceutical or pharmaceutical composition comprising an extract of flowers and/or fruits of Opuntia Ficus Indica in combination with an extract of Oryza sativa as active principles together with adjuvants and/or excipients physiologically acceptable, for use in the prevention and treatment of metabolic diseases or disorders related to inhibition of the enzymatic activity of the 5-alpha reductase, selected from the group consisted by benign prostatic hypertrophy, or androgenic alopecia. The extracts of flowers and/or fruits of Opuntia Ficus Indica and Oryza sativa (Black rice) present in the compositions according to the invention are in a reciprocal weight ratio comprised between 1:1 and 1:25, preferably 1:10 or 1:20.

According to the present invention some hybrid varieties of Black rice can be used.

The extracts from flowers or from fruits of Opuntia Ficus Indica can be used alternatively to, or in combination with, each other.

According to a preferred embodiment of the present invention the pharmaceutical or nutraceutical compositions based on a combination of plant extracts of Opuntia Ficus Indica flowers and/or fruits and Oryza sativa (Black rice) for use according to the invention are devoted to the use in the treatment of benign prostatic hypertrophy. Preferably, such compositions are suitable for oral administration, still preferably in form of oral solution, oral emulsion, oral suspension, capsule, tablet or powder. In this embodiment the total concentration of the extracts used in combination is comprised between 10-90% by weight of the total composition.

According to an alternative embodiment of the present invention the cosmetic or pharmaceutical compositions based on a combination of plant extracts of Opuntia Ficus Indica flowers and/or fruits and Oryza sativa (Black rice) for use according to the invention are devoted to the use in the treatment of androgenic.

Preferably, such cosmetic or pharmaceutical compositions are suitable for topical application, still more preferably in the form of cream, pomade, ointment, foam, lotion, gel or spray. For the topical application the preferred concentration range can vary between 0.5% and 5% (preferably 2%) by weight of the final composition. It is also possible to provide their oral administration, in the form of solution, emulsion, oral suspension, capsule, tablet or granulate. For oral administration the preferred range of total concentration of the extracts used in combination can vary between 10% and 90% by weight of the final composition.

A further subject-matter of the present invention is also a combination of an extract of flowers and/or fruits of Opuntia Ficus Indica and of an extract of Oryza sativa (Black rice) for simultaneous, sequential or separate use in the prevention and in the treatment of metabolic diseases or disorders connected with inhibition of the enzymatic activity of the 5-alpha reductase. Said metabolic diseases or disorders correlated to inhibition of the enzymatic activity of the 5-alpha reductase are selected from benign prostatic hypertrophy, or androgenic alopecia.

The combination of plant extracts of Opuntia Ficus Indica flowers and/or fruits and Oryza sativa (Black rice) for use according to the invention can be suitable for oral administration or for oral application in the cosmetic, pharmaceutical or nutraceutical field.

The extracts of flowers and/or of fruits of Opuntia Ficus Indica and Oryza sativa (Black rice) are however administered in combination maintaining a reciprocal weight ratio comprised between 1:1 and 1:25, preferably a reciprocal weight ratio 1:10 or 1:20.

For oral administration the preferred range of total concentration of extracts can vary between 10% and 90% by weight of the final composition; for topical administration the preferred concentration range can vary between 0.5% and 5% by weight (preferably 2% by weight) of the final composition.

The present invention will be now described in an illustrative, but not limitative manner, according to some preferred embodiments with particular reference to the attached figures, wherein:
- Figure 1 illustrates the percentage values of cellular vitality of the BPH-1 cells (epithelial cells of benign prostatic hypertrophy) obtained after 72 hours of treatment with the extract of Opuntia flower (O); extract of Black rice (R) and their combination (O/R). *p<0.05 vs O and R;
- Figure 2 illustrates the percentage values of cellular vitality of LNCaP cells (androgen-dependent tumoral prostatic cells) obtained after 72 hours of treatment with extract of Opuntia flower (O); extract of Black rice (R) and their combination (O/R). *p<0.05 vs O and R;
- Figure 3 illustrates the percentage values of cellular vitality of DU145 cells (androgen-resistant tumoral prostatic cells) obtained after 72 hours of treatment with extract of Opuntia flower (O); extract of Black rice (R) and their combination (O/R). *p<0.05 vs O and R;
- Figure 4 illustrates the values of cellular growth vs control (not treated) of papillary dermal cells from human hair follicle (DP cells) obtained after 24 hours of treatment with vitamin C (50 µg/mL); extract of Opuntia flower (O); extract of Black rice (R) and their combination (O/R). *p<0.05;
- Figure 5 illustrates the percentages of inhibition of the 5-α-reductase enzyme obtained in BPH-1 cells after 72 hours of treatment with extract of Opuntia flower (O); extract of Black rice (R) and their combination (O/R); *p<0.05 vs O and R;
- Figure 6 illustrates the percentage values of IF/mg proteins with respect to control (expression of the radical species ROS) obtained from BPH-1 cells after 72 hours of treatment with extract of Opuntia flower (O); extract of Black rice (R) and their combination (O/R); *p<0.05 vs O and R.

With a mere illustrative, but non limitative, purpose of the present invention, hereinafter are reported studies *in vitro* carried out by the authors of the present invention on several human prostate cell lines to show the synergistic anti-proliferative effect of the compositions according to the invention containing the extracts of Opuntia ficus indica flower and of Black rice.

### EXAMPLE 1: Preparation of extracts of Opuntia ficus indica flower/fruit and of Oryza sativa

### i) Extract of Opuntia ficus indica flower/fruit

The extraction process is hereinafter described:
step a: maceration of flowers/fruits of Opuntia ficus indica (coming from cultivations of the Mediterranean basin) fresh or dried with aqueous solution, preferably acidified to pH 3, or with a hydro-alcoholic solution (contents in ethanol 10-70%) at room temperature. In order to facilitate the extraction, flowers and fruits can be crushed or chopped before contacting the extraction solvent.
step b: second extraction on the same plant matrix with a new extraction solvent, in order to facilitate recovery of the active substances present;
step c: recovery and concentration under vacuum of the extracts obtained at a temperature not higher than 50-60°C. In case of hydro-alcoholic extracts it is proceeded with the extract concentration until complete removal of the organic solvent.
step d: recovery and isolation of the active compounds through passage of the extract on a macroporous absorbing resin Amberlite XAD-7 or other similar absorbing resin, by elution with a hydro-alcoholic solution (50-90%).
step e: concentration until complete removal of the organic solvent and next drying by means of spray-drying or lyophilisation by use of a proper technological support, e.g. maltodextrins.

The extract according to the invention can be liquid or dried (powder). The extract obtained contains an amount of flavonoid compounds not lower than 0,1% w/p (range 0,1-5%) and whose contents in isorhamnetin 3-O-robinobioside is not lower than 20% with respect to the total contents of flavonoids.

### ii) Extract of Black rice (Oryza sativa L.)

The extraction process is hereinafter described:
step a: maceration of the entire grain or of the sole pigmented pericarp obtained by abrasion of the external surface of the grain with a hydro-alcoholic solution (20-60%) at acidic pH with hydrochloric acid (0,1% HCl), at room temperature.
step b: second extraction on the same plant matrix with a new extraction solvent, in order to promote recovery of the active substances present.
step c: recovery and concentration under vacuum of the extracts obtained at a temperature not higher than 50-60°C until complete removal of the organic solvent.
step d: recovery and isolation of the anthocyanoside compounds through passage of the extract on macroporous absorbing resin Amberlite XAD-7 or other similar absorbing resin, by elution with a hydro-alcoholic solution (50-90%).
step e: concentration until complete removal of the organic solvent and next drying by means of spray-drying or lyophilisation by use of a proper technological support, e.g. maltodextrins.

The invention relates to a liquid or dried (powder) extract. The extract of Black rice obtained presents an anthocyanoside contents expressed in equivalents in cyanidin-3-O-glucoside, its main constituent, comprised between 1-20% w/p.

### EXAMPLE 2: Study in vitro on the anti-proliferative activity of extracts of Opuntia ficus indica flower and of Oryza sativa

The study had as a purpose the assessment *in vitro* of the anti-proliferative activity exerted by the plant extracts of Opuntia ficus indica flower and Oryza sativa (Black rice) separately from and in combination with each other on human prostatic epithelial cells, both normal and tumoral.

The experimental protocol provided the use of a cellular model *in vitro* capable to assess the cellular vitality by MTT colorimetric assay.

### MATERIALS AND METHODS

Benign prostatic hypertrophy or hyperplasia is a disease borne by the prostatic gland. The disease is characterized by a benign increase of the gland volume due to an excessive growth of epithelial cells and of stromal elements. The malignant degeneration of such a tissue hyperplasia determines the development of a prostatic cancer.

The anti-proliferative activity of the extract from flowers of Opuntia and of Black rice and of their combination has been assessed on the following model cells of benign prostatic hypertrophy and of the prostatic cancer:
- BPH-1: epithelial cells of benign prostatic hyperplasia;
- LNCaP: androgen-dependent prostatic cancer cells;
- DU145: androgen-resistant prostatic cancer cells.

These cells express different degrees of invasiveness of prostatic diseases, from benign prostatic hypertrophy (BPH-1) to the more serious cancer forms (LNCaP e DU145).

The BPH-1 and LNCaP cells have been cultivated in medium RPMI 1640 supplemented with fetal calf serum 10%, 1 mM of glutamine and 10 µl/ml of streptomycin/penicillin. The DU145 cells have been cultivated in medium DMEM supplemented with FCS 10%, streptomycin-penicillin 1%, glutamine 1%, nonessential amino acids 1%. The cultures have been kept in an atmosphere of CO₂ 5% at 37°C. The culture medium has been changed every 2-3 days.

The BPH-1, DU145, and LnCap cells have been then plated on wells for the MTT assays and, after 24 hours of incubation in an atmosphere of CO₂ 5% at 37°C, have been treated for 72 hours with each extract used separately and in combination, according to what is reported in the following Table 1.

**TABLE 1. Extracts and their combination used in the assessment in the assay of cellular proliferation (MTT cellular assay) on BPH-1, LNCaP and Du145 cells.**

| **Sample** | **Composition** | **Concentration** |
|---|---|---|
| O | Extract of Opuntia ficus Indica flowers | 10 µg/ml |
| R | Extract of Black rice | 200 µg/ml |
| O/R | Extract of Opuntia ficus Indica flowers/extract of Black rice | 210 µg/ml |

| | | |
|---|---|---|
| O: Extract of Opuntia ficus Indica flowers; R: extract of Black rice; O/R: combination of the two extracts | | |

The cellular vitality after treatment has been measured through the colorimetric assay of tetrazolium salts, assessing the capability of the cells to reduce, by means of mitochondrial succinate dehydrogenase, 3-(4,5-dimethylthiazol-2-yl)-2,5-diphenyl tetrazolium bromide (MTT). MTT enters the cells and passes into mitochondria, wherein it is reduced to a coloured and insoluble product, which is formazan. To make the colour visible, the coloured granules of formazan are solubilized by addition of DMSO. The MTT-cleavage reaction requires full integrity of the cell and it is proportional to the its metabolic activity degree. After incubation of the cells for three hours, in 5% CO₂ at 37°C, with 20 µl of tetrazolium salt solution, solubilized in medium (5 mg/ml), and 180 µl of medium, the supernatant has been removed and 100 µl of DMSO have been added. Next, the optical density (O.D.) has been measured at a wave length of 570 nm through a spectrophotometer for micro-plate reading (Titertek Multiscan, Flow Laboratories).

The cellular vitality has been expressed as percentage of optical density with respect to not treated control. For each sample the experiment has been carried out in triplicate.

### RESULTS

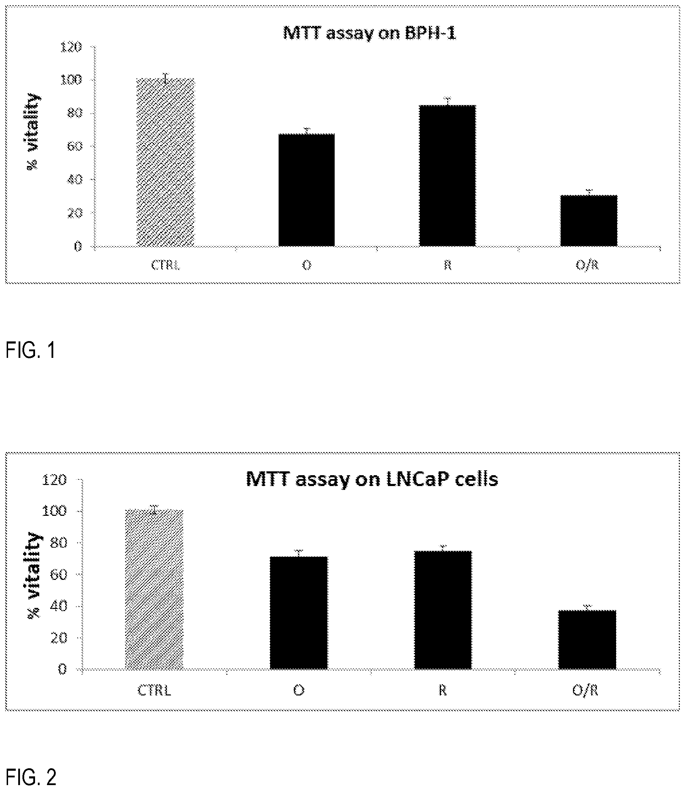

The data obtained from the performed MTT assays have shown that both the extract of Opuntia Indica flower (O) and the extract of Black rice (R) are capable to induce, when used separately, a reduction of cellular vitality on the BPH-1, LNCaP and DU145 cell lines (Table 2). The treatment of the same cell lines with both the extracts used in combination (O/R), determines a significant lowering of cellular vitality with respect to the single extracts, with an anti-proliferative effect of the synergistic type (see Figures 1-3 and Table 2). The following Table 2 illustrates the values of cellular proliferation (MTT) expressed as cellular vitality percentage with respect to control (CTRL) of epithelial cells of benign prostatic hyperplasia (BPH-1), of androgenic-dependent prostatic cancer cells (LNCaP) and androgenic-resistant prostatic cancer cells (DU145) after 72 hours of treatment.

**TABLE 2**

| | **BPH-1 Cells % cellular vitality (% inhibition)** | **LNCap Cells % cellular vitality (%inhibition)** | **DU145 cells % cellular vitality (%inhibition)** |
|---|---|---|---|
| Opuntia ficus indica flowers extract (O) | 67,42 ± 3,83 (32.6) | 71,27 ± 3.68 (28.7) | 79,91 ± 2,65 (20.1) |
| Black Rice extract (R) | 84,81 ± 4,23 (15.2) | 74,70 ± 3,46 (25.3) | 80,56 ± 3,11 (19.44) |
| Opuntia extract/Black rice (O/R) | 30,61 ± 3,11* (69.4) | 37.51 ± 2.98* (62.5) | 38,82 ± 3,53* (60.2) |

| | | | |
|---|---|---|---|
| *p<0.05 vs O and R | | | |

### EXAMPLE 3: Assessment of the activity of the extracts of Opuntia ficus indica and of Black Rice on proliferation of papillary dermal cells

The papillary dermal cells are specialized mesenchymal cells having an important role in the vital cycle of hair.

The stimulation and proliferation of these cells promote hair growth in the anagen phase. Such cells are used as model cells in the assessment of growth promoters of the hair bulb.

### MATERIALS AND METHODS

The papillary dermal cells from a human hair follicle (DPcells, PromoCell) have been cultivated in growth medium (PromoCell) supplemented with 100 units/mL of penicillin and 100 µg/mL of streptomycin, in an atmosphere of CO₂ 5% at 37°C. The DP cells have been subsequently plated on wells for the MTT assays and, after 24 hours of incubation in an atmosphere of CO₂ 5% at 37°C, they have been treated for 24 hours with each extract used separately and in combination, according to what has been indicated in Table 1. The cellular vitality after treatment has been measured through the MTT colorimetric assay (according to the protocol above described). The vitamin C has been used as positive control (50 µg/mL).

### RESULTS

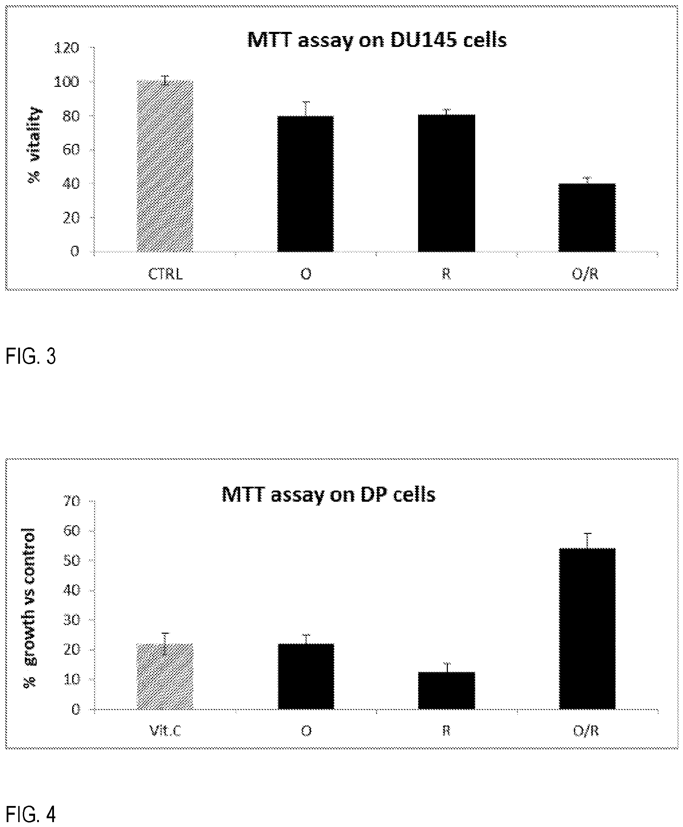

The results obtained from the cellular proliferation assay performed on papillary dermal cells from a human hair follicle show how the extract of Opuntia flower (O) and of Black rice (R) determine a moderate proliferative effect, lower than or comparable to that of vitamin C used as positive control (Figure 4). Said effect however becomes significantly higher when the two extracts are used in combination (O/R), with an increase of the growth cell of about 54%.

### EXAMPLE 4: Assessment of the mechanism of action exerted by the extracts of Opuntia ficus indica flower and of Black rice on cellular proliferation

In the present study it has been carried out an assessment of the potential mechanisms of action involved in the effect exerted by the extracts of Opuntia flower and of Black rice, used alone or in combination, using the epithelial cells of benign prostatic hyperplasia BPH-1 as model cells in the following assays:
- expression of the 5-α-reductase enzyme through the Western-blot technique (enzymatic inhibition);
- production of radicalic species of the ROS oxygen (antioxidant activity);
- expression of the Akt/PKB factor through the ELISA technique (cell cycle regulation).

### MATERIALS AND METHODS

### Cell culture and treatment

The BPH-1 cells have been cultivated in medium RPMI 1640 supplemented with FCS 10%, 1 mM of glutamine and 10 µl/ml of streptomycin/penicillin and kept in an atmosphere of CO₂ 5% at 37°C. The culture medium has been changed every 2-3 days. Twenty four hours before the experiments, the cells have been trypsinized, counted in a hemocytometer, seeded into new wells and treated for 72 hours with each extract, used separately and in combination, according to what is reported into the following Table 3.

**TABLE 3. Extracts and their combination used in the assays of 5α-reductase inhibition, of ROS production and cellular cycle regulation on BPH-1 cellular culture.**

| **Sample** | **Composition** | **Concentration** |
|---|---|---|
| O | Opuntia ficus indica flower extract | 10 µg/ml |
| R | Black rice extract | 200 µg/ml |
| O/R | Opuntia extract/Black rice | 210 µg/ml |

### Inhibition of the 5-α-reductase enzyme

The BPH-1 cells treated have been cold washed twice with PBS and then collected with a lysis buffer containing 10 mM of Tris-HCl, 10 mM of KCl, 2 mM of MgCl₂, 0,6 mM of PMSF, and 1% of SDS with a pH 7.4. After cooling for 10 min at 0°C, the proteins have been collected after centrifugation. Sixty micrograms of total proteins, present in the supernatant, have been loaded in each track and then separated by electrophoresis in polyacrylamide gel. Subsequently, the proteins have been transferred in a nitrocellulose membrane in a moist environment.

The unspecific sites of membranes have been masked by incubation with a saline buffer containing 0,01% of Tween-20 (TBST) and 5% of fat-free milk powder at 4°C for a whole night.

Antibodies directed against the 5α-reductase properly diluted in TBST, have been incubated for 2 hours at room temperature and detected with a secondary antibody conjugated with peroxidase using the substrate Supersignal West Pico Chemioluminescent (Pierce Chemical Co., Rockford, IL) for chemo-luminescence. The expression of proteins has been quantified by means of densitometric analysis of autoradiographs. The density of the single bands for each sample has been expressed in relation to that of α-tubulin, taken as referenced protein, and the values indicated (correspondent to signal intensity) have been reported as arbitrary densitometric units. From these data it has been calculated the inhibition percentage with respect to control for each sample. Each experiment has been carried out in triplicate.

### RESULTS

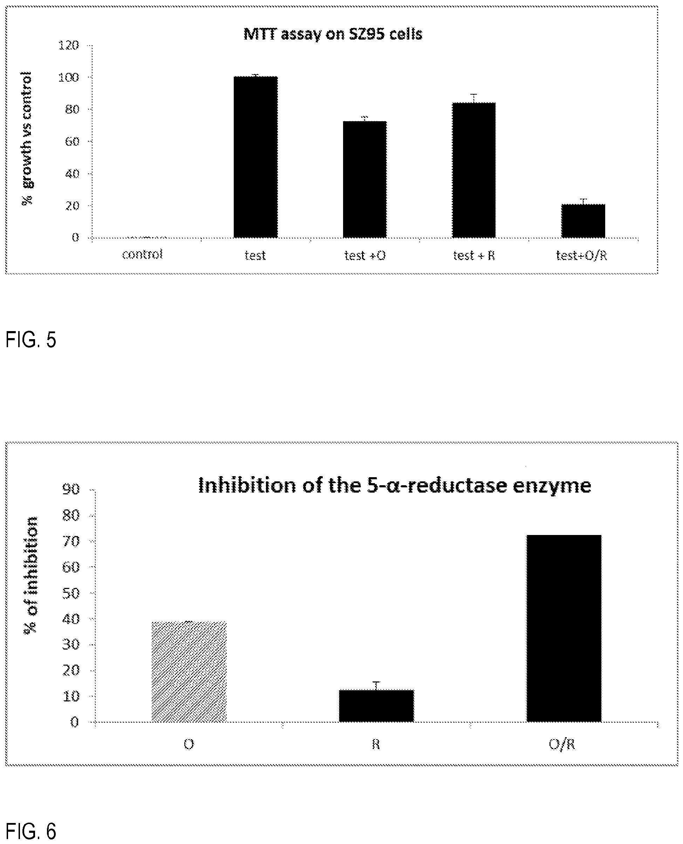

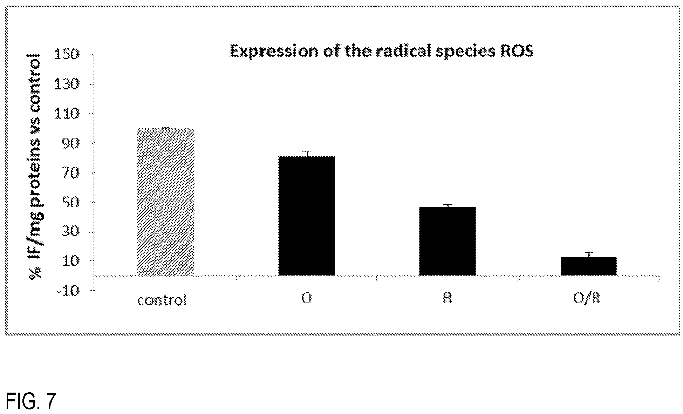

The data obtained from the studies carried out have shown that the extract of Opuntia presents a greater inhibition of the expression of the 5α-reductase enzyme with respect to the extract of Black rice, with a percentage reaching about 40% (see Table 4; Figure 5).

**TABLE 4. Percentages of inhibition of the 5α-reductase enzyme obtained from epithelial cells of benign prostatic hyperplasia (BPH-1) treated with extract of Opuntia flower (O) and of Black rice (R) alone or in combination (O/R).**

| **Sample** | **% of inhibition vs control** |
|---|---|
| *Opuntia* ficus indica flower extract (O) | 39,01 ± 3,26 |
| Black rice extract (R) | 12,46 ± 2,52 |
| *Opuntia* extract/Black rice (O/R) | 75,48 ± 2,36* |

| | |
|---|---|
| *p<0.05 vs O and R | |

However, when the extracts are used in combination, it is observed a considerable strengthening of this effect with an inhibition percentage equal to about 75%.

### Determination of the radicalic species of oxygen (ROS)

For determination of ROS it has been used dichlorofluorescein diacetate (DCFH-DA) not fluorescent. In the presence of ROS, the DCFH-DA hydrolyzes and oxidizes to DCF, which is a highly fluorescent molecule. The intensity of intracellular fluorescence of DCF has been used to quantify the oxidizing species present in the cell using a fluorometer Hitachi at λEx =485 nm and λE =530 nm.

The results have been expressed as fluorescence intensity/mg of proteins.

### RESULTS

The data obtained from the experiment have shown that the treatment of the BPH-1 cellular line with the extract of Black rice determines a lower expression of the radicalic species ROS and thus an antioxidant protection effect higher than that obtained from the extract of Opuntia flower (see Table 5 and Figure 6). Moreover, when the cells have been treated with both the extracts, the production of radicalic species results to be considerably reduced (87% inhibition), with a synergistic effect between the two extracts in strengthening the intracellular antioxidant defences.

**TABLE 5. Percentage values vs control of the radicalic species ROS expressed by the BPH-1 cells treated with the extract of Opuntia flower (O) and of Black rice (R) alone and in combination O/R.**

| **Sample** | **% IF/mg protein vs control (% inhibition)** |
|---|---|
| Opuntia ficus indica flower extract (O) | 81,16 ± 3,53 (18,8%) |
| Black rice extract (R) | 46,07 ± 2,79 (53,9%) |
| *Opuntia* extract/Black rice (O/R) | 12,95 ± 2,90* (87,1%) |

| | |
|---|---|
| *p<0.05 vs O and R. | |

### Determination of the Akt/PKB factor expression

Determination of the expression of the fosfo-AKT(Thr308) and fosfo-AKT(Ser473) on lysate of the BPH-1 cells has been performed through STAR (Signal Transduction Assay Reaction) kit ELISA, according to the specification of the supplier. The colorimetric determination has been performed by measuring the absorbance at a wave length of 450nm and the contents in p-Akt(Thr308) and p-Akt(Ser473) has been expressed in U/ng Akt total.

### RESULTS

In the cellular samples treated with the extract of Opuntia flower and Black rice it is observed a significant reduction of the Akt(Thr308) and Akt(Ser473) levels with respect to not treated cells used as control (Table 6). This effect results however to be considerably strengthened when the extracts are used in combination (O/R).

**TABLE 6. Expression of the Akt(Thr308) and Akt(Ser473) factors in BPH-1 cells treated with the extract of Opuntia flower (O) and of Black rice (R) alone and in combination (O/R)**

| **Treatment** | **Akt(Thr308) U/ng Akt total** | **Akt(Ser473) U/ng Akt total** |
|---|---|---|
| Control | 0.89 ± 0.018 | 0.085 ± 0.004 |
| Opuntia flower extract (O) | 0.78 ± 0.023 | 0.070 ± 0.003 |
| Black rice extract (R) | 0.80 ± 0.015 | 0.077 ± 0.003 |
| Opuntia extract/ Black rice (O/R) | 0.48 ± 0.010* | 0.051 ± 0.002* |

| | | |
|---|---|---|
| *p<0.05 vs O and R | | |

The several experimental models adopted in EXAMPLES 2-3 allowed to assess the effect of the extracts of Opuntia indica flower and of Black rice, used alone or in combination, also in the treatment of several androgenic-dependent diseases such as: benign prostatic hypertrophy (in different degrees of aggressiveness) through the BPH-1, LNCaP and DU145 cellular lines; androgenic alopecia through the papillary dermal cells from human hair follicle (DPcells).

The MTT assay of cellular proliferation has been adopted as assessment and quantification method on each of the cellular lines used. The data obtained have shown how the single extracts are capable to act as proliferation regulators of the cellular lines adopted. They indeed inhibit hyper-proliferation expressed by the BPH-1, LNCaP and DU145 prostatic cellular lines and promote vitality of the hair papillary dermal cells. Such effects are significantly strengthened on all the cellular lines when the two extracts are used in combination, with a synergistic type action.

### EXAMPLE 5: Examples of formulations

Hereinafter are exemplified several formulations for oral use (tablets, capsules, powders) and for topical use (gel, tonic) comprising the combination of the extract of Opuntia Indica flower/fruit and of Black rice with a different reciprocal weight ratio.

### I) Examples of formulations for oral use

**- CAPSULES (1:10 ratio)**

| **COMPONENTS** | **% w/p** |
|---|---|
| Opuntia flowers extract | 1% |
| Black rice extract | 10% |
| Lacto se | Up to 100 gr |

### Method of preparation:

Mix the extracts with lactose, according to the geometric dilution rules then proceed with repartition in the capsules.

**- TABLETS (1:20 ratio)**

| **COMPONENTS** | **% w/p** |
|---|---|
| Opuntia flowers extract | 4.2% |
| Black rice extract | 84.2% |
| Vegetal Magnesium stearate | 0.7% |
| Silicon dioxide | 1.4% |
| Talc | 0.7% |
| Gum Arabic | 0.7% |
| Citric acid | 0.8% |
| Coating agents: | |
| - Modified pregelatinized starch | 3.5% |
| - Talc | 2.2% |
| - Shellac | 0.9% |
| - Glycerol | 0.7% |

### Method of preparation:

Mix the extracts of Black rice and Opuntia ficus indica flower and proceed, according to practice, to fluid bed granulation, using a granulating solution acidified with citric acid and gum arabic.

Mix the granulate with the anti-caking agents (talc, silicon dioxide, magnesium stearate); subject the mixture to compression by means of a compressing machine. Proceed with a primary coating of the tablets with shellac and talc and a subsequent coating with modified pregelatinized starch, glycerol and talc.

**- POWDER FOR ORAL USE (example of 1:1 ratio)**

| **COMPONENTS** | **% w/p** |
|---|---|
| Opuntia fruit extract | 5% |
| Black rice extract | 5% |
| Silicon dioxide | 0.67% |
| Sucralose | 0.17% |
| Maltodextrin | Up to 100 gr |

### Method of preparation:

Mix the single components with maltodextrin, according to the geometric dilution rules, then proceed to the repartition in sachet or bags.

### II) Examples of formulations for topical use

**- ANTI-HAIR LOSS TONIC**

| **COMPONENTS** | **% w/p** |
|---|---|
| Opuntia flowers extract | 0.05% |
| Black rice extract | 0.5% |
| Ethanol | 18.2% |
| Chlorphenesin, methylparaben | 0.3% |
| Fragrance (perfume) | 0.05 |
| Citric acid 10% | a sufficient amount |
| Deionized water | Up to 100 gr |

### Method of preparation:

Solubilize the extracts in water acidified with citric acid, adding ethanol and the other components under mechanical stirring and at room temperature.

Filtrate for removing possible suspended particles.

**- GEL**

| **COMPONENTS** | **% w/p** |
|---|---|
| Hydroxyethylcellullose (Natrosol) | 1.5% |
| Opuntia fruit extract | 0.1% |
| Black rice extract | 2% |
| Propylenglycol | 10% |
| Sodium citrate | 0.35% |
| Sodium metabisulfite | 0.50% |
| Disodium EDTA | 0.10% |
| Methyl p-hydroxybenzoate | 0.20% |
| Propyl p-hydroxybenzoate | 0.05% |
| Deionized water | Up to 100 gr |

### Method of preparation:

Solubilize sodium citrate in water and subsequently all the other ingredients except for hydroxyethylcellulose (Natrosol). Add to the solution obtained hydroxyethylcellulose in portions, in order to promote its dispersion, and leaving to stir for about 12 hours for the formation of gel. Verify that the pH of the formulate does not exceed a value equal to 5.

### BIBLIOGRAPHIC REFERENCES

(1) Azzouni F. et al. Advances in Urology. 2012, pages 1-18.
(2) Arias-Santiago S. J. Am. Acad. Vol. 66, No. 3, pages 401-408.
(3) Edwards J. American Family Physician Vol. 77, Number 10, May 2008.
(4) Iperplasia Prostatica Benigna. AURO.it, pp. 1-48, 2004. Redazione Zadig.
(5) Chun H.M. et al. Asian Journal of Andrology. Vol.15, pp. 471-482, 2013.
(6) McPartland J.M. & Pruitt P.L. JAOA. Vol. 100 Number 2, February 2000.
(7) Marberger M. Adv. Ther. Vol. 30(4), pages: 309-319, 2013.
(8) US Patent Application No. 2013/0259958.
(9) Park H.J. World J. Mens Health. Vol. 31(3), pages 193-207, 2013.
(10) Izumi K. The American Journal of Pathology. Vol. 182(6), June 2013.
(11) Beduschi MC & Oesterling JE. Mayo Clin Proc. Vol. 73(7), pp:696-701, July 1998.
(12) Teng J. et al. BJU Int. Vol. 111(2), pages:312-23, February 2013.
(13) Bienovà M. et al. Acta Dermatoven APA. Vol. 14, No. 1, pages 1-8, 2005.
(14) Jonas A. et al. Urol. Res. Vol. 26, pages 265-270, 1998.
(15) Ammar I. et al. Industrial Crops and Products. Vol. 37, pages 34-40, 2012.
(16) De Leo M. et al. Phytochemistry Letters. Vol.3, pages 48-52, 2010.
(17) Deng G.F. et al. Crit Rev Food Sci Nutr. Vol. 53(3), pages 296-306, 2013.
(18) Min-Kyoung K. et al. Nutrition Research and Practice Vol. 2(1), pages 46-49, 2008.

## Claims

1. Nutraceutical or pharmaceutical composition comprising a combination of an extract of Opuntia ficus indica flowers and/or fruits and an extract of Oryza sativa (Black rice) as active principles, together with pharmaceutically acceptable adjuvants and/or excipients, for use in the prevention and treatment of diseases or disorders related to the inhibition of the enzymatic activity of the 5-alpha reductase selected between benign prostatic hyperplasia or androgenic alopecia.

2. Nutraceutical or pharmaceutical composition for use according to claim 1, wherein the extract of Opuntia ficus indica flowers and/or fruits and the Oryza sativa (Black rice) extract are in a reciprocal weight ratio comprised between 1:1 and 1:25, preferably 1:10 or 1:20.

3. Nutraceutical or pharmaceutical composition for use according to anyone of claims 1-2, for use in the prevention and treatment of benign prostatic, which is suitable for the oral administration.

4. Nutraceutical or pharmaceutical composition for use according to claim 3, in the form of oral solution, oral emulsion, oral suspension, capsule, tablet, powder or granulate.

5. Pharmaceutical composition for use according to anyone of claims 1-2, for use in the prevention or treatment of androgenic alopecia, which is suitable for topical or oral administration.

6. Pharmaceutical composition for use according to claim 5, suitable for the topical application in the form of cream, pomade, ointment, foam, lotion, gel or spray.

7. Composition suitable for the oral administration for use according to anyone of claims 3-5, wherein the total concentration of the extracts is comprised between 10%-90% in weight percentage of the final composition.

8. Pharmaceutical composition suitable for the topical application for use according to anyone of claims 5-6, wherein the total concentration of the extracts is comprised between 0.5%-5% in weight percentage of the final composition.

9. A combined preparation of an extract of Opuntia ficus indica flowers and/or fruits and an extract of Oryza sativa (Black rice) for simultaneous or separate use in the prevention or treatment of diseases or disorders related to the inhibition of the enzymatic activity of the 5-alpha reductase, selected between benign prostatic hyperplasia or androgenic alopecia.

10. A combined preparation for use according to claim 9, suitable for topical or oral administration.

## Patentansprüche

1. Nutrazeutische oder pharmazeutische Zusammensetzung, umfassend eine Kombination aus einem Extrakt von Blüten und/oder Früchten von Opuntia ficus indica und einem Extrakt von Oryza sativa (Schwarzer Reis) als Wirkstoffe, zusammen mit pharmazeutisch unbedenklichen Adjuvantien und/oder Hilfsstoffen, zur Verwendung bei der Prävention und Behandlung von Krankheiten oder Störungen, die mit der Hemmung der Enzymaktivität der 5-alpha-Reduktase in Zusammenhang stehen und aus gutartiger Prostatahyperplasie oder androgenetischer Alopezie ausgewählt sind.

2. Nutrazeutische oder pharmazeutische Zusammensetzung zur Verwendung nach Anspruch 1, wobei der Extrakt von Blüten und/oder Früchten von Opuntia ficus indica und der Extrakt von Oryza sativa (Schwarzer Reis) in einem gegenseitigen Gewichtsverhältnis vorliegen, das zwischen 1:1 und 1:25 liegt, vorzugsweise 1:10 oder 1:20.

3. Nutrazeutische oder pharmazeutische Zusammensetzung zur Verwendung nach einem der Ansprüche 1 bis 2, zur Verwendung bei der Prävention und Behandlung von gutartiger Prostata, die zur oralen Verabreichung geeignet ist.

4. Nutrazeutische oder pharmazeutische Zusammensetzung zur Verwendung nach Anspruch 3, in Form von oraler Lösung, oraler Emulsion, oraler Suspension, Kapsel, Tablette, Pulver oder Granulat.

5. Pharmazeutische Zusammensetzung zur Verwendung nach einem der Ansprüche 1 bis 2, zur Verwendung bei der Prävention oder Behandlung von androgenetischer Alopezie, die zur topischen oder oralen Verabreichung geeignet ist.

6. Pharmazeutische Zusammensetzung zur Verwendung nach Anspruch 5, die zur topischen Anwendung in Form von Creme, Pomade, Salbe, Schaum, Lotion, Gel oder Spray geeignet ist.

7. Zusammensetzung, die zur oralen Verabreichung geeignet ist, zur Verwendung nach einem der Ansprüche 3 bis 5, wobei die Gesamtkonzentration der Extrakte zwischen 10%-90% in Gewichtsprozent der endgültigen Zusammensetzung liegt.

8. Pharmazeutische Zusammensetzung, die zur topischen Anwendung geeignet ist, zur Verwendung nach einem der Ansprüche 5 bis 6, wobei die Gesamtkonzentration der Extrakte zwischen 0,5%-5% in Gewichtsprozent der endgültigen Zusammensetzung liegt.

9. Kombinationspräparat aus einem Extrakt von Blüten und/oder Früchten von Opuntia ficus indica und einem Extrakt von Oryza sativa (Schwarzer Reis) zur gleichzeitigen oder getrennten Verwendung bei der Prävention oder Behandlung von Krankheiten oder Störungen, die mit der Hemmung der Enzymaktivität der 5-alpha-Reduktase in Zusammenhang stehen und aus gutartiger Prostatahyperplasie, oder androgenetischer Alopezie ausgewählt sind.

10. Kombinationspräparat zur Verwendung nach Anspruch 9, das zur topischen oder oralen Verabreichung geeignet ist.

## Revendications

1. Composition nutraceutique ou pharmaceutique comprenant une combinaison d'un extrait de fleurs et/ou de fruits d'Opuntia ficus indica et d'un extrait d'Oryza sativa (riz noir) comme principes actifs, conjointement à des adjuvants et/ou excipients pharmaceutiquement acceptables, pour l'utilisation dans la prévention et le traitement de maladies ou de troubles liés à l'inhibition de l'activité enzymatique de la 5-alpha réductase sélectionnées parmi l'hypertrophie bénigne de la prostate ou l'alopécie androgénique.

2. Composition nutraceutique ou pharmaceutique pour l'utilisation selon la revendication 1, dans laquelle l'extrait de fleurs et/ou de fruits d'Opuntia ficus indica et l'extrait d'Oryza sativa (riz noir) sont dans un rapport de poids réciproque compris entre 1:1 et 1:25, de préférence 1:10 ou 1:20.

3. Composition nutraceutique ou pharmaceutique pour l'utilisation selon l'une quelconque des revendications 1-2, pour l'utilisation dans la prévention et le traitement de bénigne de la prostate, qui est adaptée pour l'administration orale.

4. Composition nutraceutique ou pharmaceutique pour l'utilisation selon la revendication 3, sous la forme de solution orale, d'émulsion orale, de suspension orale, de capsule, de comprimé, de poudre ou de granulé.

5. Composition pharmaceutique pour l'utilisation selon l'une quelconque des revendications 1-2, pour l'utilisation dans la prévention ou le traitement de l'alopécie androgénique, qui est adaptée pour l'administration topique ou orale.

6. Composition pharmaceutique pour l'utilisation selon la revendication 5, adaptée pour l'application topique sous la forme de crème, pommade, onguent, mousse, lotion, gel ou aérosol.

7. Composition adaptée pour l'administration orale pour l'utilisation selon l'une quelconque des revendications 3 à 5, dans laquelle la concentration totale des extraits est comprise entre 10%-90% en pourcentage en poids de la composition finale.

8. Composition pharmaceutique adaptée pour l'application topique pour l'utilisation selon l'une quelconque des revendications 5 et 6, dans laquelle la concentration totale des extraits est comprise entre 0,5%-5% en pourcentage en poids de la composition finale.

9. Préparation combinée d'un extrait de fleurs et/ou de fruits d'Opuntia ficus indica et d'un extrait d'Oryza sativa (riz noir) pour l'utilisation simultanée ou séparée dans la prévention ou le traitement de maladies ou de troubles liés à l'inhibition de l'activité enzymatique de la 5-alpha réductase, sélectionnées parmi l'hypertrophie bénigne de la prostate ou l'alopécie androgénique.

10. Préparation combinée pour l'utilisation selon la revendication 9, adaptée pour l'administration topique ou orale.
